# EUROPEAN PATENT APPLICATION

(11) **EP 2 810 940 A1**
(43) Date of publication of application: **10.12.2014**
(21) Application number: 13170788.7
(22) Date of filing: 06.06.2013
(51) Int. Cl.: C07D 311/72, C07B 63/00

(54) **A Process to Prepare A Tocopherol Concentrate**

(71) Applicant: Sulzer Chemtech AG, 8404 Winterthur (CH)
(72) Inventor: Fässler, Peter, 4123 Allschwil (CH)
(74) Representative: Manitz, Finsterwald & Partner GbR

(57) **Abstract**

A process to prepare a concentrate comprising a tocopherol compound is disclosed. The process comprises the following sequence of steps: (i) an evaporation step 10 in a first evaporator 20 of a feed stream 30 comprising a a fatty acid or its ester, and a tocopherol compound 1, wherein a first residue stream 50 and a first volatile stream 60 are separated in the evaporation step 10, (ii) a first distillation step 70 in a first distillation apparatus 80 of the first volatile stream 60 to remove a second residue stream 90 and a second volatile stream 100 and to form a volatile product concentrate stream 110, characterized in that the first evaporator 20 is a thin film or short-path evaporator, the distillation apparatus 80 comprises at least two columns 81, 82, and wherein the concentration of the tocopherol compound 1 in the feed stream is from 1 to 30 % on a weight basis. The present invention also relates to a process to prepare said concentrate from a vegetable oil 160 and an apparatus 1000 for said process to prepare said concentrate from said vegetable oil. [FIG. 1]

## Description

### BACKGROUND OF THE INVENTION

The present invention relates to a process to prepare a concentrate comprising a tocopherol compound. The present invention also relates to a process to prepare said concentrate from a vegetable oil and an apparatus for said process to prepare said concentrate from said vegetable oil.

Processes to prepare concentrates of tocopherol are of commercial interest. Alpha, beta, gamma, and delta tocopherol (hereafter referred to as tocopherols) can be found in various ratios and concentrations in crude vegetable oils. These tocopherols are a valuable constituent of vegetable oil as they help prevent oxidation and spoilage. Tocopherols are used industrially as food additives for both animals and humans, as food conservers and antioxidants, and in cosmetic formulations. During the refining of vegetable oils a large fraction of the tocopherols are lost to various by-products and waste streams. These waste and by-products streams include deodorizer distillates, steam refining distillates, and acidulated soapstocks. The vegetable oil refining by-products typically contain from less than 1% to greater than 20% tocopherol by weight. Thus these oil refining by-products are a valuable source of raw material for the production of natural vitamin E and other tocopherol antioxidants. However, the by-product streams also generally contain substantial amounts by weight of free fatty acids; sterols; sterol esters of fatty acid; mono-, di-, and triglycerides; hydrocarbons; and several other compounds, in addition to tocopherols. For this reason, in order to obtain a tocopherol concentrate stream useful for production of high purity vitamin E, it is necessary to remove these substances.

Process for the production of tocopherol concentrate streams are known. For example, US 5,512,691 discloses a state of the art process based on an esterification reaction to convert volatile alcohols to their less volatile fatty acid esters, followed by a series of distillation steps in which components having higher or lower boiling points are separated from the tocopherols and other similar boiling substances.

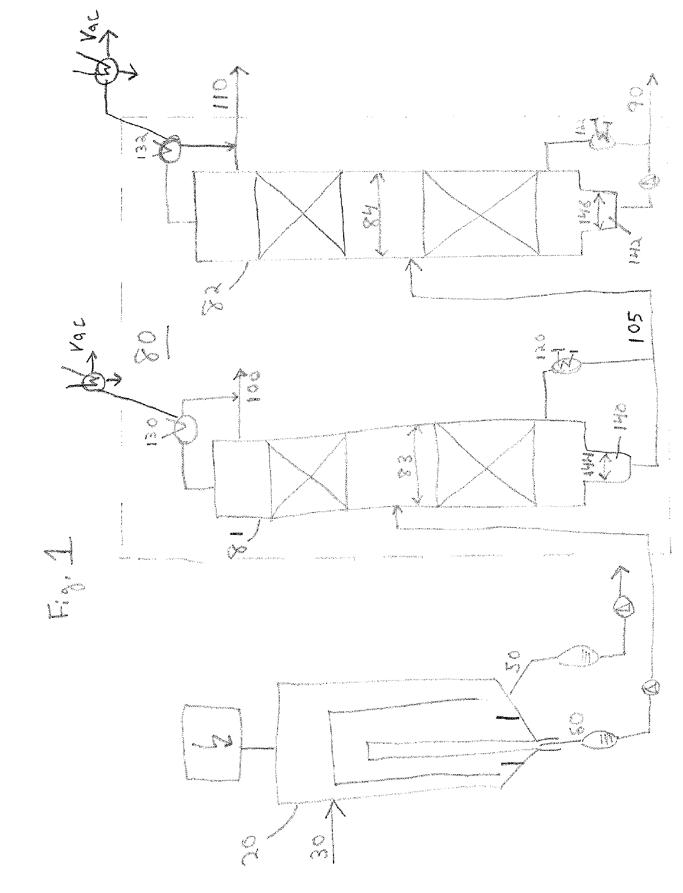

Nonetheless this disclosed process of US '691 has numerous shortcomings. Various arrangements of equipment are disclosed for carrying out the series of distillation operations, but all of the disclosed arrangements have been found to suffer from various disadvantages as described later in our comparative examples. For example, using the disclosed series of three columns in three subsequent distillation operations (Fig. 1 and modeled using ASPEN™ run in Example 15 of US '691) has been found to require in actual practice quite high bottom pressures in the columns, particularly the first column. These relatively high bottom pressures then require relatively high operating temperatures resulting in undesired excessive thermal stress, which then leads to extensive degradation of the tocopherol concentrate, as evidenced by its dark yellow to brown color.

Alternatively it is disclosed that a distillation column may be used in a first distillation operation, followed by a subsequent distillation operation in an evaporator or centrifugal molecular still, as in Example 16 of US '691. However we have found that such arrangements also lead to highly discolored concentrate products because of a poor separation process. In particular, the removal of color bodies was found to be insufficient. Without wishing to be bound to any particular mechanism, such color bodies may potentially include carotenoids, chlorophyll, and oxidized polyunsaturated compounds.

Another arrangement disclosed in US '691 is a first distillation operation in an evaporator followed by a second distillation operation in a column, as in Examples 17 and 18 of US '691. We have found such arrangements to yield tocopherol concentrates having poor quality and color, as well as containing significant amounts of heavies.

Yet another arrangement disclosed and claimed in US '691 consists of a series of three distillations carried out in a series of three evaporators and/or centrifugal molecular stills. Such arrangements are disadvantageously complex and expensive to design and operate.

It is noted that the ability to economically produce tocopherol concentrates of a high purity and good color is of importance for both synthetic vitamin E, as tocopherolacetate, and natural vitamin E, as tocopherols and tocotrienols. For example, for pharmaceutical, food and cosmetic applications, tocopherol concentrates are generally required to have quite high purities (≥ 99.0 wt %) while being close to odorless and with only a pale yellow color.

The production of natural tocopherol concentrates with such high purity and good properties is quite demanding and complex. For example, in the case of tocopherolacetate (synthetics), the feed stock concentration for the final purification is quite high, typically 85 to 93 wt % tocopherols; however, the feedstock for natural vitamin E typically starts with a very low concentration of only about 2000 to 10000 ppm tocopherols. Furthermore many vegetable oils, such as the increasingly industrially-important palm oil, have significant contents of carotenoids, which have intense orange colors and readily thermally degrade to give a variety of intensely colored degradation products. Therefore the production of natural concentrates requires quite extensive and challenging concentration and purification. Unfortunately the thermally-based separation methods such as those of US '691 have been found generally to produce only highly discolored thermally-stressed and degraded concentrate products when the required high purities are obtained on an industrial scale.

In conclusion, it would be desirable to have a process to prepare concentrates of tocopherols having high purities together with low odor and color properties, especially when starting from vegetable oils as raw material, particularly those such as palm oil containing significant quantities of carotenoids.

### SUMMARY OF THE INVENTION

Starting from this state of the art, it is an object of the invention to provide a process to prepare a concentrate comprising a tocopherol compound that does not suffer from the previous mentioned deficiencies, particularly a high thermal stress thereby decreasing product yield and quality. Further objects of the invention include providing a process to prepare said concentrate from a vegetable oil and an apparatus for said process to prepare said concentrate from said vegetable oil.

According to the invention, these objects are achieved by a process to prepare a concentrate comprising a tocopherol compound, wherein the process comprises the following steps in the following sequence:
(i) an evaporation step in a first evaporator of a feed stream comprising (a) a fatty acid or its ester and (b) a tocopherol compound, wherein a first residue stream and a first volatile stream are separated in the evaporation step, wherein the first volatile stream comprises the fatty acid or its ester and the tocopherol compound;
(ii) a first distillation step in a first distillation apparatus of the first volatile stream to remove a second residue stream and a second volatile stream comprising the fatty acid or its ester, and to form a volatile product concentrate stream comprising the tocopherol compound.

According to the invention, these further objects are achieved firstly by a process wherein the feed stream has been produced in a feed stream production process comprising the steps in the following sequence:
(i) a physical refining step of a vegetable oil to form a first fatty acid distillate comprising a free fatty acid and the tocopherol compound,
(ii) removing a first portion of the free fatty acid from the first fatty acid distillate stream by means of a second distillation step in a second distillation apparatus to form a second fatty acid distillate stream and a third residue stream comprising a remaining portion of the free fatty acid and the tocopherol compound,
(iii) esterifying substantially all of the remaining portion of the free fatty acid in the third residue stream in an esterification step to form an esterified product stream comprising a fatty acid ester, a glyceride, and the tocopherol compound,
(iv) transesterifying the glyceride of the esterified product stream in a transesterification step to form a transesterified product stream,
(v) removal of a fatty acid ester distillate from the transesterified product stream by means of a third distillation step in a third distillation apparatus to form the feed stream.

The other further object to provide an apparatus for said process to prepare said concentrate from said vegetable oil is achieved by an apparatus comprising the following sequence of units in fluid communication with each other:
(i) a stripping column for a physical refining of a vegetable oil 160 to form a first fatty acid distillate comprising a free fatty acid and a tocopherol compound,
(ii) a second distillation apparatus for removing a first portion of a free fatty acid from the fatty acid distillate to form a second fatty acid distillate stream 210 and a third residue stream comprising a remaining portion of the free fatty acid and the tocopherol compound,
(iii) an esterification reactor for esterifying substantially all of the free fatty acid in the third residue stream in an esterification step to form an esterified product stream,
(iv) a transesterification reactor for transesterifying the glyceride in the esterified product stream to form a transesterified product stream,
(v) a third distillation apparatus for removing a fatty acid ester distillate from the transesterified product stream to form a feed stream comprising (a) a fatty acid or its ester and (b) the tocopherol compound,
(vi) a first evaporator which is a thin film or short-path evaporator for separating a first residue stream and a first volatile stream, wherein the first volatile stream comprises the fatty acid or its ester and the tocopherol compound , and
(vii) a first distillation apparatus comprising a first and a second column in fluid communication with each other and for the distillation of a first volatile stream to remove a second residue stream and a second volatile stream comprising the fatty acid or its ester, and to form a volatile product concentrate stream comprising the tocopherol compound.

The present invention achieves these objects and provides a solution to this problem by means of a first evaporator which is a thin film or short-path evaporator, and a first distillation apparatus comprises a first and a second column in fluid communication with each other, and wherein the concentration of the tocopherol compound in the feed stream is from 1 to 30, preferably 2 to 25, more preferably 3 to 20 % on a weight basis.

These results surprisingly allow a thermal separation to be effected with both a high product yield and quality in terms of low odor and color. Without wishing to be bound by a particular mechanism, the inventor believes that entering the first evaporator with a minimum tocopherol content together with the use of a short film evaporator for the initial heavies removal allows the subsequent removal of light and heavy boilers to be effected in a first and second column operating under relatively mild conditions.

In one embodiment of the process, the tocopherol compound is selected from the group consisting of a tocopherolacetate, a tocopherol, a tocotrienol and their mixtures. As discussed earlier, such compounds and their mixtures are particularly sensitive to thermal stress and degradation, and thus they benefit greatly from the present invention.

According to another embodiment of the process, the number of theoretical stages in the first distillation apparatus are between 15 and 30 and a bottom pressure in the first and second columns is less than or equal to 2.5, preferably 2, more preferably 1.5, most preferably 1 mbar (abs). These parameters of number of theoretical stages and bottom pressure have been found to be particularly important for minimizing the thermal stress in the process, thereby increasing both yield and improving the product quality. Likewise in embodiments of the process starting with the physical refining of a vegetable oil, maintaining a bottom pressure in the second distillation apparatus and/or third distillation apparatus of less than or equal to 12, preferably 10, more preferably 8, most preferably 6 mbar (abs) provides similar benefits in minimizing thermal stress.

The number of theoretical stages in the present application is determined by means of the Fenske equation based on running an appropriate test mixture (for example, as disclosed in Recommended Test Mixtures for Distillation Columns, Ed. F.J. Zuiderweg, publ. by Institute of Chemical Engineers, London, 1969) together with computer simulation (e.g. Aspen Plus Dynamics^{®}).

The bottom pressure in a column in the present application may be measured by capacitive, Pirani, piezo, piezoresistive, or mechanical methods. It is noted that mechanical methods are the most robust but the least accurate; whereas the other methods have high precision but are susceptible to fouling. In the present application, bottom pressure in a column is measured in a lower portion of the column, e.g. below the last packed bed or preferably in the boot, by means of a capacitive pressure measurement device. Pressure measurement methods are described in "Instrumentation and Control for the Chemical, Mineral, and Metallurgical Processes" by V. R. Radhakrishnan, Allied Publishers, India, 1997 (ISBN : 81-7023-723-8).

According to yet another embodiment of the process and the apparatus, each of the first and second columns is equipped with a second and third evaporator and a first and second condenser, wherein the pressure drop in each one of the second and third evaporators and each one of the first and second condensers is less than 0.2, preferably 0.1, more preferably 0.05 mbar. The use of the evaporators and condensers with the columns, as well as maintaining a low pressure drop in them, has been found to be important in allowing a milder thermal separation to be effected. In a more specific embodiment, each one of the second and third evaporators is a falling film evaporator or a wiped film evaporator and each one of the condensers is a column overhead condenser or cold trap. Both types of evaporators have a favourable smooth operation also characterized by a lack of super-heating of the fluids together with a reduced liquid hold-up in them. Such characteristics are important for minimizing the thermal stress and thereby increasing yield and improving the product quality. It is noted that the wiped film evaporator has the least amount of liquid holdup.

The pressure drop in an evaporator or condenser in the present application is defined as the difference in the pressures measured in the inlet versus the outlet of the evaporator or condenser, and it is measured by means of a capacitive pressure measurement device.

In yet another embodiment, each of the first and second columns is equipped with a first and second boot having a reduced first and second boot diameter relative to the first and second shell diameters of the first and second columns. The provision of columns with boots having reduced diameters relative to their respective column diameters has been found to beneficially reduce thermal stress in the process by limiting liquid holdup and therefore the liquid residence time.

According to yet a further embodiment of the apparatus and the process, the flange connections of the first evaporator, the first distillation apparatus, and the piping in fluid connection with the first evaporator and the first distillation apparatus having a diameter of at least 3 inches, preferably at least 2 inches are either tongue and groove flanges or lip seal welded flanges or their combinations. The use of such flanges has been found to provide a high tightness against air leakage, which greatly benefits the product color and purity as many of the components are quite sensitive to oxidation, particularly at elevated temperatures. Preferably such flanges will be used for the entire apparatus or plant, and flanges will be avoided as much as possible.

In embodiments of the process starting from the physical refining of a vegetable oil, the esterification step may be carried out in a continuous process using an immobilised heterogeneous catalyst. Benefits of these embodiments include a minimization of corrosion due lack of acidic homogeneous catalysts, lack of additional chemical raw materials (catalysts and neutralizers), no neutralization of acidic catalysts is required, and high catalyst recovery. Benefits of this embodiment for the product tocopherol concentrate include a lack of contamination of the product by acid catalysts or additional components such as neutralizing agents, which may be quite important for regulatory approval, such as from the US FDA.

According to still yet a further other embodiment of the process, the esterification step and the transesterication step are carried out using methanol or ethanol, preferably bioethanol, as reactants. These alcohols and their esters are readily handled, separated and recycled, as appropriate. The use of bioethanol improves the environmental friendliness of the process, as well as maintains a high biocontent in the various products.

According to still yet further embodiments of the process, the feed stream is a residue stream obtained from the distillation of a stream of fatty acid ester, preferably biodiesel, or it is a stream of a fatty acid ester, preferably biodiesel. As the process of the invention is well suited for dealing with tocopherols and their vegetable oil raw materials with their typical impurities such as carotenoids and/or chlorophyll, such feed streams are well suited for this process.

One skilled in the art will understand that the combination of the subject matters of the various claims and embodiments of the invention is possible without limitation in the invention to the extent that such combinations are technically feasible. In this combination, the subject matter of any one claim may be combined with the subject matter of one or more of the other claims. In this combination of subject matters, the subject matter of any one process claim may be combined with the subject matter of one or more other process claims or the subject matter of one or more apparatus claims or the subject matter of a mixture of one or more process claims and apparatus claims. By analogy, the subject matter of any one apparatus claim may be combined with the subject matter of one or more other apparatus claims or the subject matter of one or more process claims or the subject matter of a mixture of one or more process claims and apparatus claims. By way of example, the subject matter of any one claim may be combined with the subject matters of any number of the other claims without limitation to the extent that such combinations are technically feasible.

One skilled in the art will understand that the combination of the subject matters of the various embodiments of the invention is possible without limitation in the invention. For example, the subject matter of one of the above-mentioned apparatus embodiments may be combined with the subject matter of one or more of the other above-mentioned process embodiments or vice versa without limitation so long as technically feasible.

### BRIEF DESCRIPTION OF THE DRAWINGS

The invention will be explained in more detail hereinafter with reference to various embodiments of the invention as well as to the drawings. The schematic drawings show:
- Fig. 1: shows a schematic view of an embodiment of a first evaporator and a first distillation apparatus for preparing a concentrate comprising a tocopherol compound.
- Fig. 2: shows a schematic view of an embodiment of a process for preparing a concentrate comprising a tocopherol compound starting from a physical refining of a vegetable oil.
- Fig. 3: shows a schematic view of an embodiment of an apparatus suitable for a process to preparing a concentrate comprising a tocopherol compound starting from a physical refining of a vegetable oil.
- Fig. 4: shows the chemical structures of some tocopherol compounds.

### DETAILED DESCRIPTION OF THE INVENTION

### DEFINITIONS

As used in the specification and claims of this application, the following definitions, should be applied:
"a", "an", and "the" as an antecedent refer to either the singular or plural. For example, "a tocopherol compound" refers to either a single species of compound or a mixture of such species unless the context indicates otherwise.

"substantially all" as in "esterifying substantially all of the remaining portion of the free fatty acid" refers to the reaction being carried out to essentially full conversion. In one embodiment the reaction is carried out such that less than 1000, preferably 500 ppm of unconverted species remain as determined by gas chromatographic analysis, and in a preferred embodiment the unconverted species is no longer detectable. For example newly revised European method EN 14103 provides standard GC methods for quantification of Fatty Acid Methyl Esters (FAME). Further information on quantitative GC methods is found in "Quantitative Gas Chromatography for Laboratory Analyses and On-Line Process Control", by G. Guiochon, C.L. Guillemin, published by Elsevier, Amsterdam 1988 (ISBN: 978-0-444-42857-8).

A "tocopherol compound" is any of several closely related compounds, found in wheatgerm oil, egg yolk, and leafy vegetables etc., which collectively constitute vitamin E. They are fat-soluble alcohols with antioxidant properties, important in the stabilization of cell membranes. The tocophenol compounds are a series of organic compounds consisting of various methylated phenols, and they may exist in eight different forms, four tocopherols and four tocotrienols. All feature a chromanol ring, with a hydroxyl group that can donate a hydrogen atom to reduce free radicals and a hydrophobic side chain which allows for penetration into biological membranes. Both the tocopherols and tocotrienols occur in alpha, beta, gamma and delta forms, as determined by the number and position of methyl groups on the chromanol ring. Thus the tocopherol compound of the present invention may be selected from α-Tocopherol, β-tocopherol, γ-Tocopherol, δ-Tocopherol, α-Tocotrienol, β-Tocotrienol, γ-Tocotrienol, δ-Tocotrienol, α-Tocomonoenol, β-Tocomonoenol, γ-Tocomonoenol, δ-Tocomonoenol, α-MDT, β-MDT, γ-MDT, δ-MDT and their various mixtures.

The term "tocophenol compound" of the present invention encompasses also synthetic forms of compounds having vitamin E activity such as alpha tocopheryl acetate as a single stereoisomer or as a mixture of any two or more of its eight stereoisomers. In one embodiment it is the 8-isomer *all-rac* vitamin E commonly known as dl-tocopherol or dl-tocopheryl acetate, but actually dl,dl,dl-tocopherol. The term "tocopherol compound" of the present invention also encompasses the ester derivates common in the industry, prepared by converting the phenol form of the vitamin E active compound (with a free hydroxyl group) to an ester, for example using acetic or succinic acid. These tocopheryl esters are more stable and are easy to use in vitamin supplements. As other examples of industrially important esters, tocopheryl nicotinate and tocopheryl linolate esters are also used in cosmetics and some pharmaceuticals (for example, as disclosed in Wikipedia: http://en.wikipedia.org/wiki/Tocopherol).

Numerical values in the present application relate to average values. Furthermore, unless indicated to the contrary, the numerical values should be understood to include numerical values which are the same when reduced to the same number of significant figures and numerical values that differ from the stated value by less than the experimental error of the conventional measurement technique of the type described in the present application to determine the value.

FIG. 1 shows a schematic view of a first evaporator 20 and a first distillation apparatus 80 for preparing a concentrate comprising a tocopherol compound 1.

The first evaporator 20 is a thin film or short-path evaporator, and it is used for carrying out an evaporation step 10 of a feed stream 30 comprising (a) a fatty acid or its ester and (b) a tocopherol compound 1. The concentration of the tocopherol compound 1 in the feed stream 30 is from 1 to 30, preferably 2 to 25, more preferably 3 to 20 % on a weight basis, and the feed stream 30 is otherwise not specifically limited. Feed stream 30 may contain a variety of optional additional components such as C6 - C24 fatty acids, mono,di- or triglycerides, carotenoids, squalenes, sterols, waxes, and various unsaponifiable components.

As an example, the feed stream 30 may be a residue stream obtained from the distillation of a stream of fatty acid ester, preferably biodiesel, or it may be a stream of a fatty acid ester, preferably biodiesel. For example, palm fatty acid distillate, considered a non-edible side product in the refining process of palm oil, contains typically about 3000 ppm of a mixture of tocopherols and tocotrienols. Alternatively, the feedstream 30 may originate from a physical refining of a vegetable oil 160 such as a wheat germ oil, palm kernel oil, coconut oil, olive oil, cocoa butter, peanut oil, oat oil, safflower oil, soya bean oil, sunflower oil, rice bran oil, barely oil, or palm oil, as will be described in detail later.

In this evaporation step 10, a first volatile stream 60 comprising the fatty acid or its ester and the tocopherol compound 1 are separated from a first residue stream 50. The content of the tocopherol compound 1 in the first volatile stream 60 will typically be between about 5 to about 25 wt. %, preferably at least about 10, more preferably at least about 20 wt. %. Other components typically will include remaining FAME's, fatty acids, monoglycerides, squalenes, sterols, and heavy neutrals. The residue stream 50 comprises heavies such as fatty acids, traces of monoglycerides, squalenes, sterols, di-and triglycerides, and heavy neutrals. Typically the residue stream 50 contains about 0.5 to about 10 wt % of tocopherol compounds.

Evaporators and their construction and operation are well known in the art, for example, as disclosed in Handbook of Evaporation Technology, by P.E. Minton, published in 1986 by Noyes (ISBN 0-8115-1097-7), Fundamentals and modeling of separation processes: absorption, distillation, evaporation and extraction, by C.D. Holland, published in 1975 by Prentice-Hall (ISBN 0-13-344390-6), and Selecting Evaporators For Process Applications, by W.B. Glover, Chemical Engineering Progress, Dec. 2004, p. 26 - 33, www.cepmagazine.org. Unless indicated otherwise, conventional construction materials and means, as well as components and auxiliaries, may be used for the evaporator 20, and the evaporator 20 may be operated in an evaporation process in a conventional manner as known in the art. For example, these cited reference handbooks and textbooks disclose a variety of conventional means for evaporating, heat exchanging and condensing for use in evaporators. The process operating conditions in the first evaporator 20 will generally be a pressure of less than 0.5, preferably 0.3, more preferably 0.1, and most preferably 0.001 mbar (abs), and a temperature of generally less than or equal to 250, preferably 230, more preferably 210, most preferably 180°C.

Also shown in FIG. 1 is an embodiment of the first distillation apparatus 80 comprising a first and a second column, 81, 82, in fluid communication with each other. Distillation columns and their construction and operation are well known in the art, for example, as disclosed in Chemical Engineering Design, Vol. 6, Coulson & Richardson's Chemical Engineering Series, by R. K. Sinnott, John Metcalfe Coulson, and John Francis Richardson, 4th Ed. Published in 2005 by Elsevier (ISBN 0 7506 6538 6). Unless indicated otherwise, conventional construction materials and means, as well as components and auxiliaries, may be used for the first and a second column, 81, 82, and they may be operated in a distillation in a conventional manner as known in the art, for example, in previously cited textbooks, unless indicated otherwise.

As shown in FIG. 1, each of the first and second columns, 81 and 82, is preferably equipped with a second and third evaporator, 120 and 122, and a first and second condenser, 130 and 132. The process operating conditions in the first and second columns, 80 and 81, will preferably include a bottom pressure of less than or equal to 2.5, preferably 2, more preferably 1.5, most preferably 1 mbar (abs) and a bottom temperature of generally less than or equal to 250, preferably 240, more preferably 230 °C. When second and third evaporators, 120 and 122, and first and second condensers, 130 and 132, are present, their pressure drops will preferably be less than 0.2, more preferably 0.1, most preferably 0.05 mbar. As shown, it may often be preferable to have a cold trap on the vacuum line from the condenser to the vacuum system to remove non-condensables.

Typical components of the second volatile stream 100 from first column 81 include FAME's, monoglycerides, squalenes, tocopherols, and carotenoids. Typical components of the 105 residue stream from first column 81 include traces of monoglycerides, squalenes, tocopherols, sterols, di- and triglycerides, and heavy neutrals. Typical components of the volatile product concentrate stream 110 from second column 82 include enriched tocopherols, traces of monoglycerides, squalenes, sterols and heavy neutrals. Typical components of the second residue stream 90 from second column 82 include traces of squalenes, tocopherols, mono-, di- and triglycerides, and heavy neutrals.

Also shown in the embodiment of FIG. 1 is the embodiment in which each of the first and second columns, 81 and 82, is equipped with a first and second boot, 140 and 142, having a reduced first and second boot diameter, 144 and 146, relative to their first and second shell diameters, 83 and 84.

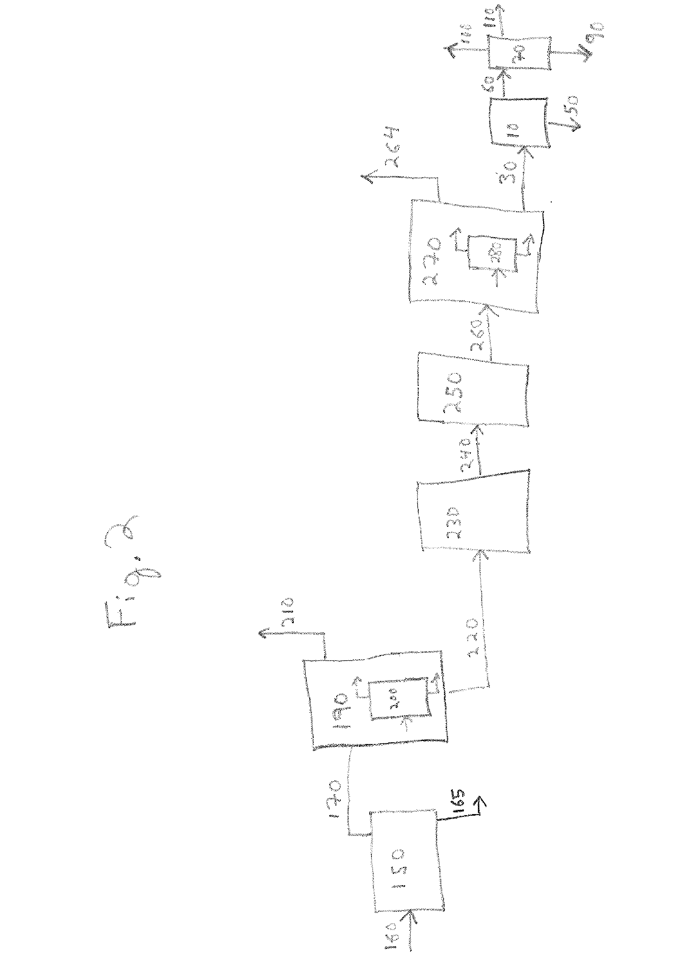

FIG. 2 shows a schematic view of an embodiment of a process for preparing a concentrate comprising a tocopherol compound 1 starting from a physical refining of a vegetable oil 160. In this process the feed stream 30 has been produced in a feed stream 30 production process comprising the following steps in the following sequence:
First, a physical refining step 150 of a vegetable oil 160 to form a first fatty acid distillate stream 170 comprising a free fatty acid and the tocopherol compound 1 and a residue stream 165 typically comprising refined vegetable oil. Other typical components of the vegetable oil 160 include hydrocarbons, sterol esters of fatty acids, sterols, triterpenoid alcohols, methyl-sterols, and mono-, di-, and triglycerides. The fatty acid is a carboxylic acid with a long aliphatic tail (chain), which may be either saturated or unsaturated. "Free fatty acid" refers to a fatty acid in its non-esterified acidic form. Typically the chain contains an even number of carbon atoms, preferably from 4 to 28. Examples of unsaturated fatty acids include Myristoleic acid, Palmitoleic acid, Sapienic acid, Oleic acid, Elaidic acid, Vaccenic acid, Linoleic acid, Linoelaidic acid, α-Linolenic acid, Arachidonic acid, Eicosapentaenoic acid, Erucic acid, and Docosahexaenoic acid. Examples of saturated fatty acids include Caprylic acid, Capric acid, Lauric acid, Myristic acid, Palmitic acid, Oleic acid, Linoleic acid, Linolenic acid, Stearic acid, Arachidic acid, Behenic acid, Lignoceric acid, and Cerotic acid. Other typical components of the first fatty acid distillate stream 170 include mono-, di-, and triglycerides. Typical operating conditions for the physical refining step 150 include an inlet temperature of 250 - 265 °C and a top pressure of about 2 - 5 mbar (abs).

Second, removing a first portion of the free fatty acid from the first fatty acid distillate stream 170 by means of a second distillation step 190 in a second distillation apparatus 200 to form a second fatty acid distillate stream 210 and a third residue stream 220 comprising a remaining portion of the free fatty acid and the tocopherol compound 1. Typical and preferred operating conditions for the second distillation step 190 include a bottom pressure in the second distillation apparatus 200 of less than or equal to 12, preferably 10, more preferably 8, most preferably 6 mbar (abs), and a temperature of less than or equal to 230, preferably 220 °C. The second fatty acid distillate stream 210 will generally comprise a substantially pure fatty acid mixture of C6 to C22 fatty acids together with low amounts of glycerides and moisture. The third residue stream 220 will generally comprise about 30 to about 50 wt % fatty acids with the rest of the components the same as those of first fatty acid distillate stream 170.

Third, esterifying substantially all of the remaining portion of the free fatty acid in the third residue stream 220 in an esterification step 230 to form an esterified product stream 240 comprising a fatty acid ester, a glyceride, and the tocopherol compound 1. The fatty acid ester may be an alkyl, phenyl or aralkyl ester, preferably it will be a methyl or ethyl ester. The esterification step will generally take place in the presence of a catalyst. For example, the homogeneous catalysts, esterification process and conditions disclosed in US 5,512,691 may be used. The free fatty acid reacts with the sterols to form high-boiling sterol esters and water. Any other alcoholic moieties (including added alcohols such as methanol or ethanol), triterpenoid alcohols, methyl-sterols, and the like, also react with the free fatty acids to form high-boiling fatty acid esters, waxes, and water. Removal of the water of esterification drives the reaction equilibrium toward the formation of the fatty acid esters, and more efficient catalyst types and increased catalysts loading, reaction time and temperature may be generally used to esterify substantially all of the free fatty acid. Generally the stoichiometric excess alcohol and reaction water will be removed as part of the esterification step.

Preferably the esterification step 230 is carried out in a continuous process using an immobilized heterogeneous catalyst. Suitable methods for the esterification of fatty acids are disclosed in US 7,091,367 B2. Heterogeneous catalysts are categorized as solid acid and solid base. Solid base catalysts include a wide group of compounds such as alkaline earth metal hydroxides, hydrotalcites/layered double hydroxides, alumina loaded with various compounds, zeolites, and various other compounds showing high basicity coupled with active basic sites and pore size. Solid acid catalysts include resins such as partially-sulfonated crosslinked polystyrene, zeolites, and tungstophosphoric acid supported on inorganic oxides. Preferred immobilized heterogeneous catalysts for the esterification step 230 include sulfonated polystyrene resins such as those available under the trademark AMBERLITE.

Fourth, transesterifying the glyceride of the esterified product stream 240 in a transesterification step 250 to form a transesterified product stream 260. Glycerides are esters formed from glycerol and fatty acids. Glycerol has three hydroxyl functional groups, which can be esterified with one, two, or three fatty acids to form monoglycerides, diglycerides, and triglycerides. As in the esterification step 230, typically a catalyst and added alcohol will be used in the transesterification step 250. Preferred reaction conditions include the use of a basic catalyst such as alkali metal hydroxides, methoxides or ethoxides, particularly KOH or sodium methoxide, in a continuous process.

The objective of the esterification step 230 and transesterification step 250 is to transform undesired fatty acids and heavy and intermediate boiling compounds (relative to the desired tocopherol compounds 1) into volatile compounds such as FAME's, which may then be removed in subsequent steps to improve the purification level and yield achieved. Generally atmospheric or slight overpressures are preferred. The reaction temperatures will be as low as possible to still obtain reasonable reaction rates and keep the reactants in the liquid phase while avoiding side reactions such as the dehydration of alkyl alcohols to give their corresponding alkanes or reactions to produce diakyl ethers. Typical reaction temperatures are less than or equal to 100, preferably 80, more preferably 65, most preferably 60 °C. In a preferred embodiment, methanol is used as alcohol in these two steps and is subsequently recovered using a methanol recovery unit.

Fifth, removal of a fatty acid ester distillate 264 from the transesterified product stream 260 by means of a third distillation step 270 in a third distillation apparatus 280 to form the feed stream 30. Besides the tocopheol compound 1 to be concentrated and up to about 30% fatty acid esters, the feed stream 30 will typically comprise such additional optional components as carotenoids, squalenes, sterols, waxes, unsaponifiable components and other heavy boiling components. Typical and preferred operating conditions for the third distillation step 270 include a bottom pressure in the third distillation apparatus 280 of less than or equal to 12, preferably 10, more preferably 8, most preferably 6 mbar (abs), and a temperature of less than or equal to 230, preferably 220 °C. The third distillation apparatus may comprise two distillation columns, and, if so, the bottom pressure in the second column will preferably be less than 2 mbar (abs). The feed stream 30 is processed next in an evaporation step 10 to yield a first residue stream 50 and a first volatile stream 60. The first volatile stream 60 is then treated in a first distillation step 70 in order to remove a second residue stream 90 and a second volatile stream 100 to form a volatile product concentrate stream 110 comprising the tocopherol compound 1. It is noted that evaporation step 10 and the first distillation step 70 have been described earlier and a suitable first evaporator and a first distillation apparatus for those steps are shown, for example, in FIG. 1 and were also described earlier.

It is noted that the disclosed process for preparing a concentrate comprising a tocopherol compound 1 starting from a physical refining of a vegetable oil 160 comprising these five steps may contain additional steps so long as these five steps are carried out in this relative sequence. Thus additional steps may take place before all of the five steps, between any two of the five steps, or after the five steps or various combinations of these possibilities.

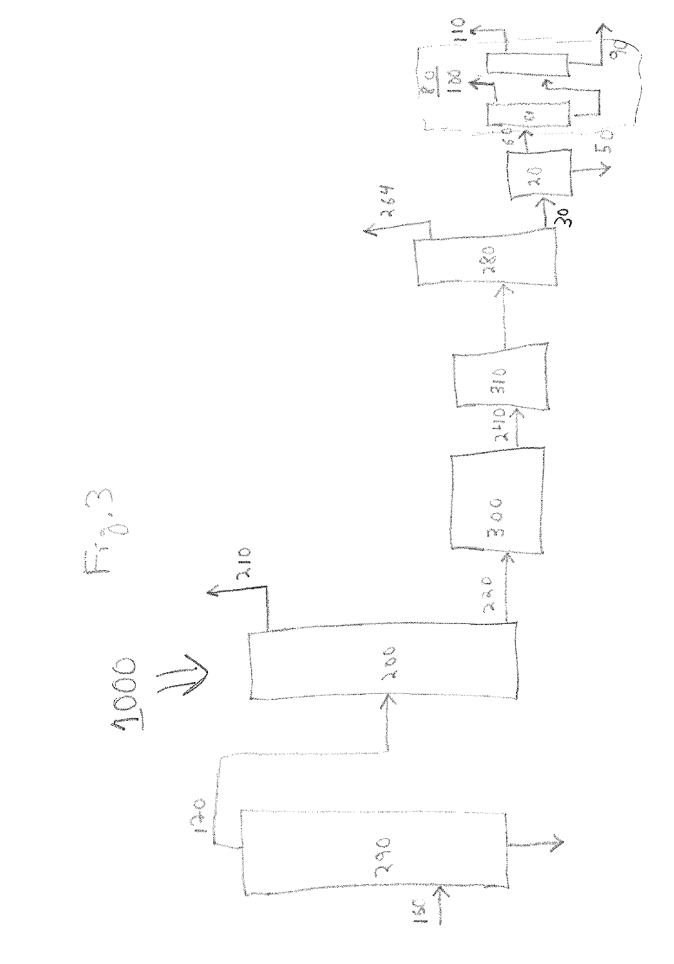

FIG. 3 shows a schematic view of an embodiment of an apparatus 1000 suitable for a process to preparing a concentrate comprising a tocopherol compound starting from a physical refining of a vegetable oil 160. The apparatus 1000 comprises the following sequence of units in fluid communication with each other:
Firstly, a stripping column 290 for a physical refining of a vegetable oil 160 to form a first fatty acid distillate 170 comprising a free fatty acid and a tocopherol compound 1. Typical and preferred operating conditions for the stripping column 290 are those disclosed earlier for the physical refining step 150.In a preferred embodiment, the stripping column comprises a wash section as disclosed in the presentation by Peter Faessler entitled "Recent Developments and Improvements in Palm Oil Stripping and Fatty Acid Distillation", at the World Conference on Palm and Coconut Oil for the 21st Century, in Denpasar, Indonesia in 1998.

Secondly, a second distillation apparatus 200 for removing a first portion of a free fatty acid from the fatty acid distillate 170 to form a second fatty acid distillate stream 210 and a third residue stream 220 comprising a remaining portion of the free fatty acid and the tocopherol compound 1.

Thirdly, an esterification reactor 300 for esterifying substantially all of the free fatty acid in the third residue stream 220 in an esterification step 230 to form an esterified product stream 240.

Fourthly, a transesterification reactor 310 for transesterifying substantially all of a glyceride in the esterified product stream 240 to form a transesterified product stream 260.

Fifthly, a third distillation apparatus 280 for removing a fatty acid ester distillate 264 from the transesterified product stream 260 to form a feed stream 30 comprising (a) a fatty acid or its ester and (b)the tocopherol compound 1.

Sixthly, a first evaporator 20 which is a thin film or short-path evaporator for separating a first residue stream 50 and a first volatile stream 60, wherein the first volatile stream 60 comprises the fatty acid or its ester and the tocopherol compound 1.

Seventhly, a first distillation apparatus 80 comprising a first and a second column, 81 and 82, in fluid communication with each other and for the distillation of a first volatile stream 60 to remove a second residue stream 90 and a second volatile stream 100 comprising the fatty acid or its ester, and to form a volatile product concentrate stream 110 comprising the tocopherol compound 1.

It is noted that the disclosed apparatus 1000 for preparing a concentrate comprising a tocopherol compound 1 starting from a physical refining of a vegetable oil 160 comprising these seven equipment units may contain additional equipment units so long as these seven equipment units are present and in fluid communication in this relative sequence. Thus additional units may be placed before all of the seven units, between any two of the seven units, or after the seven units or various combinations of these possibilities.

Apparatus 1000 may comprise auxiliaries that are conventional and well-known in the art and include electrical supplies, coolant and heating fluid supplies and distributions, level controllers, pumps, valves, pipes and lines, reservoirs, drums, tanks, and sensors for measuring such parameters as flow, temperatures, pressures and levels. The apparatus 1000 and the process of the invention may be conveniently controlled by means of a computer interface equipped with appropriate sensors.

Although not shown in the schematic figures for simplicity, one skilled in the art will understand that other conventional separation device internals may be used without limitation in the invention, such as feed devices like feed pipes and/or sumps, heat exchangers, support plates and grids, dispersers, disperser/support plates, continuous phase distributors, support and holddown plates, baffles, deflectors, entrainment separators, and retainers/redistributors.

Mass transfer internals such as random or structured packings or trays may be beneficially used in the processes and apparatus of the invention. Particularly effective have been found to be structured packing characterized by a hold-up profile above intersectional loading points that is reduced by the smooth change of flow direction of the packing structure. At the lower and upper end of each packing element, the orientation of the corrugation gradually approaches the vertical axis. This structural features suppresses the local flooding at intersection points and increases the overall packing capacity. A preferred packing of this type is the Sulzer product MellapakPlus™ structured packing based on metal sheets or more preferably BXPlus™ structured packing based on metal gauze. Such packings are disclosed in the Sulzer technical brochure Structured Packings for Distillation, Absorption and Reactive Distillation (publication 22.13.06.40 - IX.12 - 10).

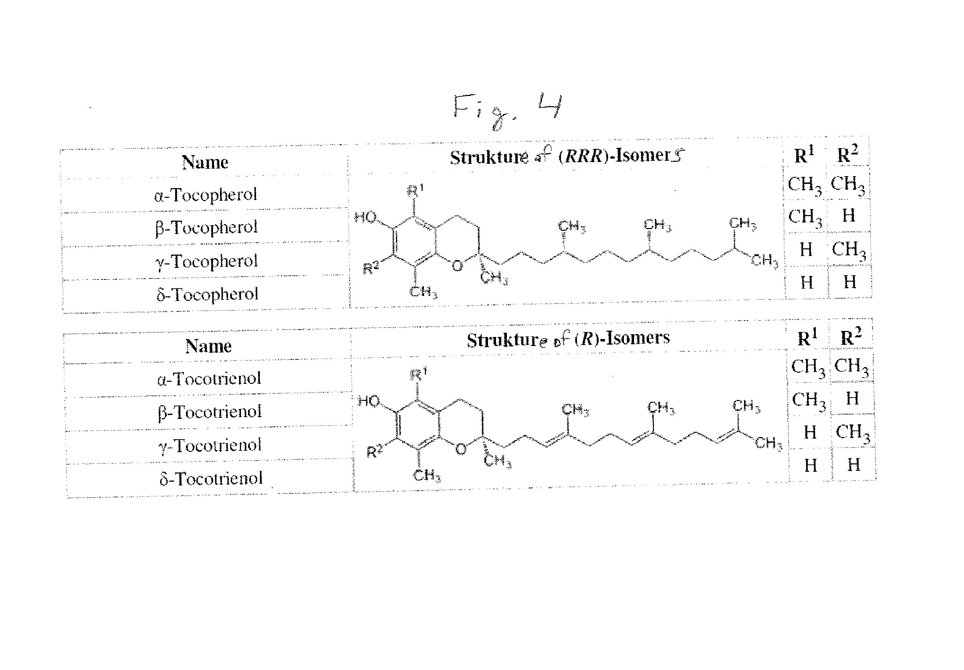

FIG. 4 shows the chemical structures of some representative tocopherol compounds.

### EXAMPLES

The following examples are set forth to provide those of ordinary skill in the art with a detailed description of how the processes to prepare a concentrate comprising a tocopherol compound 1, and apparatus 1000 and units for said process claimed herein are evaluated, and they are not intended to limit the scope of what the inventors regard as their invention.

In these examples, the process of claim 1 was evaluated such that a first evaporator 20, which was a thin film evaporator, and a first distillation apparatus 80, which comprised a first and a second column, 81 and 82, in fluid communication with each other, were used in a typical application for the preparation of a concentrate comprising a tocopherol compound 1 from a feedstream 30 comprising (a) a fatty acid or its ester and (b) a tocopherol compound 1. In these trials, a variety of feed streams 30 having a strong odor and an APHA color of more than 500 and a tocopherol concentration of between about 5 and about 20 wt % were tested in the process of the invention. In these test trials of the process of the invention, a volatile product concentrate stream 110 comprising the tocopherol compound 1 was produced, and the nearly-odorless product concentrate stream 110 was found to have an APHA color of between about 100 to about 200 and a tocopherol concentration of between about 25 and about 80 wt %. Therefore these trials demonstrate the capability of the process of the invention to prepare tocopherol concentrate streams having significantly improved color properties. The color of these concentrate streams 110 was reduced versus that of the feed streams 30 indicating that the process of the invention is effective in removing color bodies.

In a first comparative example, the concentration process was carried out using three distillation columns in series, but the color of the concentrate stream obtained was dissatisfactory (APHA color > 500). This discoloration was attributed to the inability to obtain sufficiently low bottom pressures and thus sufficiently low operating temperatures, particularly in the first column. In a second comparative example a first distillation step in an evaporator was followed by a second distillation in a distillation column. In this comparative example, a reduced bottom pressure and operating temperature was obtained in the distillation column; however, the color of the resulting tocopherol concentrate was again dissatisfactory due to the poor separation of the color bodies such as carotenoids and oxidized polyunsaturated compounds. The color of the concentrates in these comparative examples was significantly worse than that of the product concentrate streams 110 obtained in the examples using the process of the invention. This result then confirms the superiority of the process of the invention versus the processes of the prior art in removing color bodies and minimizing thermal stress when preparing concentrates of tocopherol compounds 1.

While various embodiments have been set forth for the purpose of illustration, the foregoing descriptions should not be deemed to be a limitation on the scope herein. Accordingly, various modifications, adaptations, and alternatives can occur to one skilled in the art without departing from the spirit and scope herein.

### Reference Numbers

- 1: tocopherol compound
- 10: evaporation step
- 20: first evaporator
- 30: feed stream
- 50: a first residue stream
- 60: a first volatile stream
- 70: first distillation step
- 80: a first distillation apparatus
- 90: a second residue stream
- 100: a second volatile stream
- 105: residue stream from first column 80
- 110: a volatile product concentrate stream
- 80, 81: first and second columns
- 120, 122: second and third evaporator
- 130, 132: first and second condenser
- 140,142: first and second boots
- 144, 146: first and second boot diameters
- 83, 84: first and second shell diameters
- 150: physical refining step
- 160: a vegetable oil
- 165: residue stream
- 170: first fatty acid distillate stream
- 190: second distillation step
- 200: second distillation apparatus
- 210: volatile second fatty acid distillate stream
- 220: third residue stream
- 230: esterification step
- 240: esterified product stream
- 250: transesterification step
- 260: transesterified product stream
- 264: fatty acid ester distillate
- 270: third distillation step
- 280: third distillation apparatus
- 290: stripping column
- 300: esterification reactor
- 310: transesterification reactor
- 1000: apparatus

## Claims

1. A process to prepare a concentrate comprising a tocopherol compound (1), wherein the process comprises the following steps in the following sequence:
(i) an evaporation step (10) in a first evaporator (20) of a feed stream (30) comprising (a) a fatty acid or its ester and (b) a tocopherol compound (1), wherein a first residue stream (50) and a first volatile stream (60) are separated in the evaporation step (10), wherein the first volatile stream (60) comprises the fatty acid or its ester and the tocopherol compound (1);
(ii) a first distillation step (70) in a first distillation apparatus (80) of the first volatile stream (60) to remove a second residue stream (90) and a second volatile stream (100) comprising the fatty acid or its ester, and to form a volatile product concentrate stream (110) comprising the tocopherol compound (1);
**characterized in that** the first evaporator (20) is a thin film or short-path evaporator, the first distillation apparatus (80) comprises a first and a second column (81, 82) in fluid communication with each other, and wherein the concentration of the tocopherol compound (1) in the feed stream (30) is from 1 to 30, preferably 2 to 25, more preferably 3 to 20 % on a weight basis.

2. The process of claim 1, wherein the tocopherol compound is selected from the group consisting of a tocopherolacetate, a tocopherol, a tocotrienol and their mixtures.

3. The process of either one of claims 1 to 2, wherein the number of theoretical stages in the first distillation apparatus (80) are between 15 and 30 and a bottom pressure in the first and second columns (81, 82) is less than or equal to 2.5, preferably 2, more preferably 1.5, most preferably 1 mbar (abs).

4. The process of any one of claims 1 to 3, wherein each of the first and second columns (81, 82) is equipped with a second and third evaporator (120, 122) and a first and second condenser (130, 132), wherein the pressure drop in each one of the second and third evaporators (120, 122) and each one of the first and second condensers (130, 132) is less than 0.2, preferably 0.1, more preferably 0.05 mbar.

5. The process of claim 4, wherein each one of the second and third evaporators (120, 122) is a falling film evaporator or a wiped film evaporator.

6. The process of any one of claims 1 to 5, wherein each of the first and second columns (81, 82) is equipped with a first and second boot (140, 142) having a reduced first and second boot diameter (144, 146) relative to the first and second shell diameters (83, 84) of the first and second columns (81, 82).

7. The process of an one of claims 1 to 6, wherein the flange connections of the first evaporator (20), the first distillation apparatus (80), and the piping in fluid connection with the first evaporator (20) and the first distillation apparatus (80) having a diameter of at least 3 inches, preferably at least 2 inches are either tongue and groove flanges or lip seal welded flanges or their combinations.

8. The process of any one of claims 1 to 7, wherein the feed stream (30) has been produced in a feed stream (30) production process comprising the steps in the following sequence:
(i) a physical refining step (150) of a vegetable oil (160) to form a first fatty acid distillate stream (170) comprising a free fatty acid and the tocopherol compound (1),
(ii) removing a first portion of the free fatty acid from the first fatty acid distillate stream (170) by means of a second distillation step (190) in a second distillation apparatus (200) to form a second fatty acid distillate stream (210) and a third residue stream (220) comprising a remaining portion of the free fatty acid and the tocopherol compound (1),
(iii) esterifying substantially all of the remaining portion of the free fatty acid in the third residue stream (220) in an esterification step (230) to form an esterified product stream (240) comprising a fatty acid ester, a glyceride, and the tocopherol compound (1),
(iv) transesterifying the glyceride of the esterified product stream (240) in a transesterification step (250) to form a transesterified product stream (260),
(v) removal of a fatty acid ester distillate (264) from the transesterified product stream (260) by means of a third distillation step (270) in a third distillation apparatus (280) to form the feed stream (30).

9. The process of claim 8, wherein a bottom pressure in the second distillation apparatus (200) is less than or equal to 12, preferably 10, more preferably 8, most preferably 6 mbar (abs).

10. The process of either claim 8 or 9, wherein a bottom pressure in the third distillation apparatus (280) is less than or equal to 12, preferably 10, more preferably 8 mbar (abs).

11. The process of any one of claims 8 to 10, wherein the esterification step (230) is carried out in a continuous process using an immobilised heterogeneous catalyst.

12. The process of any one of claims 8 to 10, wherein the esterification step (230) and the transesterication step (250) are carried out using methanol or ethanol, preferably bioethanol, as reactants.

13. The process of any one of claims 1 to 7, wherein the feed stream (30) is a residue stream obtained from the distillation of a stream of fatty acid ester, preferably biodiesel.

14. The process of any one of claims 1 to 7, wherein the feed stream (30)
is a stream of a fatty acid ester, preferably biodiesel.

15. An apparatus (1000) for the process of any one of claims 8 to 12,
wherein the apparatus (1000) comprises the following sequence of units in fluid communication with each other:
(i) a stripping column (290) for a physical refining of a vegetable oil (160) to form a first fatty acid distillate (170) comprising a free fatty acid and a tocopherol compound (1),
(ii) a second distillation apparatus (200) for removing a first portion of a free fatty acid from the fatty acid distillate (170) to form a second fatty acid distillate stream (210) and a third residue stream (220) comprising a remaining portion of the free fatty acid and the tocopherol compound (1),
(iii) an esterification reactor (300) for esterifying substantially all of the free fatty acid in the third residue stream (220) in an esterification step (230) to form an esterified product stream (240),
(iv) a transesterification reactor (310) for transesterifying the glyceride in the esterified product stream (240) to form a transesterified product stream (260),
(v) a third distillation apparatus (280) for removing a fatty acid ester distillate (264) from the transesterified product stream (260) to form a feed stream (30) comprising (a) a fatty acid or its ester and (b)the tocopherol compound (1),
(vi) a first evaporator (20) which is a thin film or short-path evaporator for separating a first residue stream (50) and a first volatile stream (60), wherein the first volatile stream (60) comprises the fatty acid or its ester and the tocopherol compound (1), and
(vii) a first distillation apparatus (80) comprising a first and a second column (81, 82) in fluid communication with each other and for the distillation of a first volatile stream (60) to remove a second residue stream (90) and a second volatile stream (100) comprising the fatty acid or its ester, and to form a volatile product concentrate stream (110) comprising the tocopherol compound (1).
